# EUROPÄISCHE PATENTSCHRIFT

(11) **EP 2 879 727 B1**
(45) Veröffentlichungstag und Bekanntmachung des Hinweises auf die Patenterteilung: **05.06.2019**
(21) Anmeldenummer: 13741986.7
(22) Anmeldetag: 15.07.2013
(51) Int. Cl.: A61L 27/24, A61L 27/60, A61K 38/39, A61K 9/00

(54) **KIT, DESSEN VERWENDUNG UND VERFAHREN ZUM AUFFÜLLEN VON BINDEGEWEBE DER HAUT**
KIT, USE THEREOF AND METHOD FOR FILLING CONNECTIVE TISSUE OF THE SKIN
KIT, UTILISATION DUDIT KIT ET PROCÉDÉ PERMETTANT DE REMPLIR LE TISSU CONJONCTIF CUTANÉ

(30) Priorität: 31.07.2012 DE 102012213496
(43) Veröffentlichungstag der Anmeldung: 10.06.2015
(73) Patentinhaber: Meidrix Biomedicals GmbH, 73728 Esslingen (DE)
(72) Erfinder: GRAEVE, Thomas, 70597 Stuttgart (DE)
(74) Vertreter: Schrell, Andreas
(86) Internationale Anmeldenummer: PCT/EP2013/064917
(87) Internationale Veröffentlichungsnummer: WO 2014/019842

(56) Entgegenhaltungen:
- EP-B1- 1 221 937
- WO-A1-93/17075
- WO-A1-2012/107174
- US-A1- 2009 254 104

## Beschreibung

Die Erfindung betrifft einen Kit und eine Spritze zur Verwendung in einem therapeutischen Verfahren zum intrakutanen Auffüllen des Kollagengerüsts in Bindegewebe der Haut.

Aus der DE 10 026 789 A1 sind eine Kollagen-basierte Biomatrix und Verfahren zu deren Herstellung bekannt. Es wird eine Kollagenbiomatrix aus Kollagen, das aus Rattenschwanzsehnen extrahiert wurde, hergestellt. Dazu wird die nach der Extraktion vorliegende saure Kollagenlösung in der Kälte mit serumhaltigem Puffer neutralisiert und, gegebenenfalls in Gegenwart von Zellen, zu einem Kollagengel gegossen, das später zu einer kollagenhaltigen Biomatrix geliert oder quervernetzt, das heißt aushärtet, die so als konfektioniertes zellfreies Kollagenimplantat oder zellhaltiges Kollagentransplantat mit eingebetteten Chrondrozyten erhalten werden kann.

Die US 2009/0254104 A1 offenbart Verfahren und kollagenhaltige Produkte zur Therapie von Gewebedefekten. Offenbart werden insbesondere kollagenhaltige Matrizes, die zur Rekonstruktion von beispielsweise Sehnen in einen Patienten eingeführt werden. Die Modellier- oder Formbarkeit der eingebrachten kollagenhaltigen Matrix zur Ausbildung einer gewünschten dreidimensionalen Struktur des Implantats ist allerdings in situ nur begrenzt möglich.

Die EP 1 221 937 B1 offenbart Zusammensetzungen enthaltend unlösliche Kollagenfibrillen, Zellen und Mikroträger mit einer Größe von 0,1 bis 2 mm, die über z. B. Spritzen in das zu therapierende Gewebe in flüssiger Form eingebracht werden können und dort in situ zu einer festen Matrix ausgelieren können. Eine intrakutane, möglichst schmerzfreie Applikation von modellierfähigem Kollagen ist mittels dieser grob-partikulären Zusammensetzung nicht möglich.

Die EP 0 632 820 B1 offenbart hoch konzentrierte homogenisierte Kollagenzusammensetzungen, die auch mit hoher Kollagenkonzentration in zu therapierende Gewebe eingespritzt werden können. Diese speziell aufbereiteten, hoch konzentrierten injizierbaren Kollagenzusammensetzungen sind jedoch nicht gelierfähig und daher nicht geeignet, eine modellierfähige dreidimensionale Struktur in einem zu therapierenden Gewebe auszubilden.

Es besteht daher nach wie vor ein Bedarf an Verfahren und Mitteln, die es erlauben, im Umfeld von Hauterkrankungen und Hautalterungserscheinungen auftretende, vom Patienten als unerwünscht identifizierte Hautdefekte, z. B. Falten, in verbesserter Weise zu therapieren, insbesondere in für den Patienten möglichst schonender Weise eine möglichst naturgetreue Rekonstruktion des natürlichen Hautbildes, insbesondere der Hautaußenkontur zu erreichen.

Der Erfindung liegt das technische Problem zugrunde Mittel zur Herstellung einer kollagenbasierten Matrix in situ bereitzustellen, die insbesondere zur Behandlung von Hautdefekten am menschlichen oder tierischen Körper eingesetzt werden können und womit die aus dem Stand der Technik bekannten Nachteile vermieden werden können.

Zur Lösung dieses technischen Problems stellt die Erfindung den Kit und die Spritze gemäß der unabhängigen Patentansprüche bereit.

Die vorliegende Beschreibung offenbart ein Verfahren, im Rahmen dessen aus einer flüssigen, insbesondere konzentrierten, Kollagenlösung in Verbindung mit einer flüssigen Pufferlösung eine flüssige gelierfähige Kollagenzusammensetzung erhalten wird. Diese kann direkt intrakutan in die Lederhaut eines Patienten injiziert werden, wo sie unmittelbar zu einer Kollagenmatrix aushärtet, die das Hautimplantat dann direkt in situ bildet. Dabei ermöglicht die Einbringung der flüssigen gelierenden Kollagenzusammensetzung in die Lederhaut eine dreidimensionale Formgebung, z. B. eine verbesserte Anpassung an die Außenkontur des betreffenden Körperteils. Insbesondere kann die noch flüssige Kollagenzusammensetzung direkt nach der Injektion einer Formgebung unterzogen werden, das heißt modelliert werden, das heißt dreidimensional verformt werden, bevor sie ausgeliert, das heißt aushärtet.

Das offenbarte Verfahren ist vorteilhaft insofern, als dass es das Einspritzen einer flüssigen Kollagenzusammensetzung in die zu therapierende Gewebestelle, das heißt die Lederhaut, ermöglicht und dort vor Ort an der zu therapierenden Stelle ein Ausgelieren der flüssigen Kollagenzusammensetzung stattfindet, welche unmittelbar nach dem Einspritzen noch z. B. vom Operateur, in der erwünschten Art und Weise modelliert, das heißt dreidimensional in die gewünschte Form gebracht wird. Überraschenderweise ist es möglich, durch eine mit einem sehr kleinen Innendurchmesser ausgestatteten Nadel einer Spritze eine hoch viskose, mit einer vergleichsweise hohen Kollagenkonzentration ausgestattete flüssige Kollagenzusammensetzung weitgehend schmerzfrei intrakutan dem Patienten zu verabreichen, ohne dass es zu einem Verstopfen der Nadel kommt. Dies ist insbesondere auch insofern überraschend, als dass die eingesetzte flüssige Kollagenzusammensetzung ein gelierfähiges Kollagen enthält. Bevorzugt weist die flüssige gelierfähige Kollagenzusammensetzung eine Viskosität von vorzugsweise 1 bis 9 Pa x s, insbesondere 1,2 bis 8 Pa x s, vorzugsweise 2 bis 9 Pa x s, insbesondere 3,5 bis 8 Pa x s, insbesondere 2,7 bis 7 Pa x s, insbesondere 2,8 bis 6 Pa x s, insbesondere 2,9 bis 5,5 Pa x s, auf, insbesondere für Kollagenkonzentrationen von 6 bis 10 mg/ml.

In einer besonders bevorzugten Ausführungsform ist vorgesehen, dass die flüssige gelierfähige Kollagenzusammensetzung eine Viskosität von 2 bis 9 Pa x s, insbesondere 2,5 bis 8 Pa x s, insbesondere 2,7 bis 7 Pa x s, insbesondere 2,8 bis 6 Pa x s, insbesondere 2,9 bis 5,5 Pa x s, insbesondere 2, 5 bis 3 Pa x s, für eine Kollagenkonzentration von 8 mg/ml aufweist.

In einer besonders bevorzugten Ausführungsform ist eine Viskosität der flüssigen gelierfähigen Kollagenzusammensetzung von vorzugsweise 2 bis 9 Pa x s, insbesondere 3,5 bis 8 Pa x s, insbesondere 2,7 bis 7 Pa x s, insbesondere 2,8 bis 6 Pa x s, insbesondere 2,9 bis 5,5 Pa x s, insbesondere 5,0 bis 5,5 Pa x s für eine Kollagenkonzentration von 10 mg/ml vorgesehen.

In einer besonders bevorzugten Ausführungsform ist eine Viskosität der flüssigen gelierfähigen Kollagenzusammensetzung von 1 bis 2 Pa x s, insbesondere 1,2 bis 1,4 Pa x s, insbesondere 1,25 bis 1,35 Pa x s für eine Kollagenkonzentration von 6 mg/ml Kollagen vorgesehen.

Die Viskosität wird bevorzugt gemäß des in Beispiel 3 beschriebenen Verfahrens und der darin eingesetzten Vorrichtungen bestimmt.

Das offenbarte Verfahren erlaubt die Bereitstellung einer kollagenhaltigen Biomatrix oder eines Kollagenimplantats in vivo in der Haut, vorzugsweise Lederhaut, eines Patienten mit einem hohen Anteil an nicht denaturiertem, quervernetzbarem Kollagen nativer Struktur. Insbesondere erlaubt es das offenbarte Verfahren, eine große Menge hoch konzentrierten, gelierfähigen Kollagens unmittelbar in die zu therapierende Gewebestelle einzubringen, was vorteilhafterweise zu einer besonders großen Stabilität der erhaltenen, vorzugsweise modellierten, dreidimensionalen Kollagengerüststruktur führt, die sich überdies durch eine besonders lange Haltbarkeit auszeichnet. Dadurch, dass es das offenbarte Verfahren erlaubt, die hergestellte Kollagenzusammensetzung noch in flüssiger Form an den Applikationsort, nämlich die Lederhaut, zu transportieren, sind weniger große Zugänge erforderlich. Das Verfahren erlaubt eine verbesserte minimal-invasive Chirurgie und verringert so die durch den operativen Eingriff verursachten Traumata. Es hat sich gezeigt, dass vor Ort vorhandene Hautzellen besonders gut in einer Kollagenbiomatrix proliferieren und dabei eine eigene Kollagensynthese mit hoher Rate aufweisen, wenn in der Biomatrix ein hoher Anteil an nativem Kollagen enthalten ist. Das Verfahren vermeidet daher kollagendenaturierende Maßnahmen.

Im Zusammenhang mit der vorliegenden Erfindung wird unter dem Begriff Haut das der Abgrenzung von Innen und Außen des menschlichen oder tierischen Körpers dienende flächige Organ des menschlichen oder tierischen Körpers verstanden, welches sich in Oberhaut, auch als Epidermis bezeichnet, unmittelbar darunterliegende Lederhaut, auch als Dermis, Corium oder Bindegewebe bezeichnet, und Unterhaut, auch als Subcutis bezeichnet, gliedert. Es ist vorgesehen, das Kollagengerüst des Bindegewebes der Haut, das heißt das sich in der Dermis, das heißt der Lederhaut, befindende Kollagengerüst zu rekonstituieren.

Im Zusammenhang mit der vorliegenden Erfindung wird unter "Haut" auch die Schleimhaut verstanden, insbesondere Lippen.

Im Zusammenhang mit der vorliegenden Erfindung wird unter einem "intrakutanen Injizieren" verstanden, dass die zum Injizieren verwendete Nadel der Spritze in die Lederhaut, das heißt das Bindegewebe der Haut eindringt und dort die flüssige gelierfähige Kollagenzusammensetzung innerhalb der Haut ausgebracht wird.

Die vorliegende Erfindung sieht einen Kit zur Verwendung in einem therapeutischen Verfahren zum intrakutanen Auffüllen des Kollagengerüsts in Bindegewebe der Haut eines Patienten vor, umfassend mindestens einen Behälter enthaltend eine Kollagenlösung, mindestens einen Behälter enthaltend eine neutralisierende Pufferlösung und eine Spritze geeignet zum intrakutanen Injizieren der Kollagen- und Pufferlösung, wobei die Nadel der Spritze einen Außendurchmesser, im Folgenden auch Durchmesser der Nadel, insbesondere der Nadelkanüle, bezeichnet, von 0,3 bis 0,4 mm, insbesondere 0,32 bis 0,38 mm, vorzugsweise 0,35 mm, aufweist.

Der Außendurchmesser der Nadel der erfindungsgemäß eingesetzten Spritze weist erfindungsgemäß bevorzugt einen Durchmesser von 0,3 mm auf, was 30 Gauge entspricht.

In einer weiteren bevorzugten Ausführungsform ist ein Kit der vorliegenden Erfindung vorgesehen, wobei die Spritze mindestens zwei getrennte als Kammern ausgeführte Behälter aufweist, die in Fluidverbindung mit einer in der Spritze angeordneten Mischvorrichtung und der Nadel der Spritze stehen, wobei mindestens eine erste Kammer mit der Kollagenlösung und mindestens eine davon getrennte zweite Kammer mit der neutralisierenden Pufferlösung gefüllt ist, und wobei die Nadel, insbesondere Nadelkanüle, der Spritze einen Durchmesser von 0,3 bis 0,4 mm, vorzugsweise 0,3 mm, insbesondere 0,32 bis 0,38 mm, vorzugsweise 0, 35 mm, aufweist.

In einer weiteren bevorzugten Ausführungsform ist ein Kit der vorliegenden Erfindung vorgesehen, wobei die Mischvorrichtung ein statischer Mischer ist.

Die vorliegende Erfindung kann vorsehen, dass a) die vorgenannten Behälter des Kits separat zu der Spritze vorliegen, dass b) die vorgenannten Behälter separat zu der Spritze und an die Spritze reversibel oder irreversibel anbringbar ausgebildet vorliegen oder dass c) die vorgenannten Behälter integraler Bestandteil der Spritze sind.

In einer bevorzugten Ausführungsform der vorliegenden Erfindung weist ein erfindungsgemäßer Kit neben den vorstehend ausdrücklich angegebenen Komponenten, nämlich Spritze, ggf. separat vorliegenden Kammern und Puffer- und Kollagenlösung, darüber hinaus eine Verpackung und/oder schriftliche Bedienungsanleitung, insbesondere eine solche vor, die auf die Verwendbarkeit des Kits und/oder seiner Komponenten in einem therapeutischen Verfahren zum intrakutanen Auffüllen des Kollagengerüstes im Bindegewebe der Haut, insbesondere eines Patienten mit Hautdefekten, z. B. Hautalterungserscheinungen oder Hauterkrankungen, hinweist.

Die vorliegende Erfindung sieht zudem eine Spritze zur Verwendung in einem therapeutischen Verfahren zum intrakutanen Auffüllen des Kollagengerüsts in Bindegewebe der Haut eines Patienten vor, enthaltend mindestens zwei getrennte als Kammern ausgeführte Behälter, die in Fluidverbindung mit einer in der Spritze angeordneten Mischvorrichtung und der Nadel der Spritze stehen, wobei mindestens eine erste Kammer mit einer Kollagenlösung und mindestens eine davon getrennte zweite Kammer mit einer neutralisierenden Pufferlösung gefüllt ist, wobei die Nadel der Spritze einen Durchmesser von 0,3 bis 0,4 mm, vorzugsweise 0,3 mm, insbesondere 0,32 bis 0,38 mm, vorzugsweise 0,35 mm, aufweist.

Die vorliegende Beschreibung offenbart ein Verfahren zum intrakutanen Auffüllen des Kollagengerüsts in Bindegewebe der Haut eines Patienten mittels eines Kollagengels vor, enthaltend die Schritte:
a) getrenntes Bereitstellen einer Kollagenlösung und einer neutralisierenden Pufferlösung,
b) Vermischen der Kollagenlösung und der neutralisierenden Pufferlösung, wobei eine gelierfähige Kollagenzusammensetzung erhalten wird,
c) intrakutanes Injizieren der flüssigen gelierfähigen Kollagenzusammensetzung vor deren Gelieren und
d) Formgeben der injizierten flüssigen gelierfähigen Kollagenzusammensetzung am intrakutanen Bestimmungsort, wobei das geformte Kollagengel am Bestimmungsort ausgeliert.

Zudem ist ein Verfahren offenbart, wobei in Verfahrensschritt a) die Kollagenlösung und die Pufferlösung jeweils getrennt auf eine Temperatur von 20° C bis 37° C temperiert und in Verfahrensschritt b) die temperierte Kollagenlösung und die temperierte Pufferlösung vermischt werden, wobei eine flüssige gelierfähige Kollagenzusammensetzung erhalten wird.

Auch ist ein Verfahren offenbart, wobei in Verfahrensschritt b) die miteinander vermischte Kollagen- und neutralisierende Pufferlösung, das heißt die flüssige gelierfähige Kollagenzusammensetzung, auf eine Temperatur von 20 bis 37 °C temperiert werden.

In einer weiteren bevorzugten Ausführungsform des offenbarten Verfahrens ist vorgesehen, dass die Kollagenlösung und die neutralisierende Pufferlösung, die in Verfahrensschritt a) bereitgestellt werden, bei einer Temperatur von -15 bis 19 °C, vorzugsweise von 0 bis 4 °C, insbesondere von 2 bis 19 °C, insbesondere 3 bis 18 °C, vorzugsweise 4 bis 15 °C vorliegen, bevor sie temperiert, das heißt erwärmt, werden.

In einer weiteren bevorzugten Ausführungsform ist ein Verfahren offenbart, wobei das intrakutane Injizieren gemäß Verfahrensschritt c) mittels einer Spritze, enthaltend eine Nadel mit einem Durchmesser von 0,3 bis 0,4 mm, insbesondere 0,32 bis 0,38 mm, vorzugsweise 0,35 mm, erfolgt.

In einer weiteren bevorzugten Ausführungsform ist ein Verfahren offenbart, wobei das Bereitstellen gemäß Verfahrensschritt a), das Vermischen gemäß Verfahrensschritt b) und das intrakutane Injizieren gemäß Verfahrensschritt c) in einer Spritze, insbesondere mindestens zweikammerigen Spritze, enthaltend eine Mischvorrichtung und eine Nadel mit einem Durchmesser von 0,3 bis 0,4 mm, vorzugsweise 0,3 mm, insbesondere 0,32 bis 0,38 mm, vorzugsweise 0,35 mm, erfolgt.

In einer weiteren bevorzugten Ausführungsform ist ein Verfahren offenbart, wobei das Vermischen in einem Zeitraum von maximal 5 Sekunden abgeschlossen wird.

In einer weiteren bevorzugten Ausführungsform ist ein Verfahren offenbart, wobei die Gelierung der hergestellten Kollagenzusammensetzung innerhalb von 10 Sekunden nach dem Vermischen beginnt.

In einer weiteren bevorzugten Ausführungsform ist ein Verfahren offenbart, wobei das intrakutane Injizieren und Formgeben der Kollagenzusammensetzung maximal 4 Minuten, vorzugsweise maximal 2 Minuten, dauert.

In einer weiteren bevorzugten Ausführungsform ist ein Verfahren offenbart, wobei das Vermischen von Kollagenlösung und Pufferlösung durch Ausdrücken der Lösungen aus den Kammern der Spritze und Zusammenführen der Lösungsströme in einer der Spritze zugeordneten Mischvorrichtung erfolgt, wobei aus der Mischvorrichtung die hergestellte Kollagenzusammensetzung austritt.

In einer weiteren bevorzugten Ausführungsform ist ein Verfahren offenbart und eine Spritze oder ein Kit der vorliegenden Erfindung vorgesehen, wobei die aus der eingesetzten Kollagenlösung hergestellte flüssige gelierfähige Kollagenzusammensetzung eine Konzentration von 6 bis 12 mg/ml, insbesondere 7 bis 11 mg/ml, vorzugsweise 6 bis 10 mg/ml, insbesondere 9 bis 11 mg/ml, insbesondere 7 bis 9 mg/ml, vorzugsweise 8 bis 10 mg/ml, insbesondere 8 mg/ml, aufweist.

In einer besonders bevorzugten Ausführungsform ist vorgesehen, dass die eingesetzte Kollagenlösung, die nach Mischung mit einer neutralisierenden Pufferlösung zur Bereitstellung der flüssigen gelierfähigen Kollagenzusammensetzung führt, eine Kollagenkonzentration von 8 bis 16 mg/ml, vorzugsweise 9 bis 16 mg/ml, insbesondere 8 bis 12 mg/ml, insbesondere 10 bis 15 mg/ml, vorzugsweise 9 bis 13 mg/ml, bevorzugt 9 bis 11 mg/ml, insbesondere 10 mg/ml, aufweist.

In einer weiteren bevorzugten Ausführungsform ist ein Verfahren offenbart oder ein Kit der vorliegenden Erfindung vorgesehen, wobei der pH-Wert der Kollagenlösung (bezogen auf 21 °C) 6 oder weniger beträgt.

In einer weiteren bevorzugten Ausführungsform ist ein Verfahren offenbart und eine Spritze oder ein Kit der vorliegenden Erfindung vorgesehen, wobei die Kollagenlösung, insbesondere die in Verfahrensschritt a) bereitgestellte Kollagenlösung, durch saure Extraktion ohne Einsatz enzymatischer Aktivität aus kollagenhaltigem Gewebe, insbesondere Rattenschwänzen, hergestellt wurde.

Eine derartige durch saure Extraktion, ohne Einsatz enzymatischer Aktivität aus kollagenhaltigem Gewebe hergestellte Kollagenlösung wird bevorzugt dadurch gelierfähig gemacht, dass sie mit einem neutralisierenden Agens, z. B. einer neutralisierenden Pufferlösung, vermischt wird, was aufgrund der dadurch bewirkten pH-Werterhöhung den Gelierprozess startet. Eine Kollagenlösung der vorliegenden Erfindung ist daher potentiell gelierfähig. Eine flüssige Kollagenzusammensetzung der vorliegenden Erfindung ist gelierfähig.

In besonders bevorzugter Ausführungsform ist das Kollagen der Kollagenlösung natives Kollagen.

In einer weiteren bevorzugten Ausführungsform ist ein Verfahren offenbart und eine Spritze oder ein Kit der vorliegenden Erfindung vorgesehen, wobei die Kollagenlösung, die flüssige gelierfähige Kollagenzusammensetzung oder beides zellfrei ist.

In besonders bevorzugter Ausführungsform weist das offenbarte Verfahren zumindest die folgenden Schritte auf: Getrennt voneinander bereitgestellte Kollagenlösung und Pufferlösung, die insbesondere zuvor in der Kälte gelagert waren, werden jeweils auf eine Temperatur von 20° C bis etwa 37° C, bevorzugt bis etwa 30° C, temperiert; die temperierte Kollagenlösung und temperierte Pufferlösung werden, vorzugsweise unmittelbar daran anschließend, vermischt, wobei eine flüssige gelierfähige Kollagenzusammensetzung erhalten wird, die unmittelbar nach Injektion in die Lederhaut des Patienten beginnt zu gelieren und zu einer Kollagenbiomatrix aushärten kann.

Besonders bevorzugt ist vorgesehen, dass Kollagenlösung und Pufferlösung erst bei der Applikation, das heißt besonders während der Applikation der Kollagenzusammensetzung vermischt werden und sich erst während der Applikation die zunächst noch flüssige gelierende Kollagenzusammensetzung bildet. Bevorzugt wird also die gelierende, aber noch flüssige Kollagenzusammensetzung in statu nascendi appliziert, die dann am Applikationsort, nämlich der Lederhaut, aushärten kann.

Es werden bevorzugt eine konzentrierte Kollagenlösung mit vorzugsweise einer Konzentration an Kollagen von 8 bis 16 mg/ml, vorzugsweise 9 bis 16 mg/ml, insbesondere 10 bis 15 mg/ml, bevorzugt 8 bis 12 mg/ml, insbesondere 10 mg/ml und eine Pufferlösung bereitgestellt, die ungemischt, aber in einem integralen Gebinde zusammen lagerbar sind, und zwar in an sich bekannter Weise in der Kälte, besonders bei Temperaturen von etwa 0° C bis etwa 4° C. Im Rahmen des offenbarten Verfahrens werden diese erst kurz vor deren Verwendung zur in situ-Herstellung der Kollagenbiomatrix temperiert, beispielsweise auf Zimmertemperatur, das heißt besonders auf 20° C oder mehr, oder auf Körpertemperatur, das heißt besonders etwa 30° C, insbesondere von 20 bis 37 °C.

Besonders bevorzugt erfolgt die Temperierung von Kollagenlösung und Pufferlösung gleichzeitig, und zwar besonders erst unmittelbar vor dem Vermischen und Ausbringen. Dies kann durch kurzzeitiges Lagern in einem Wärmeschrank oder gegebenenfalls durch Aufwärmen in der Hand erfolgen. Die Erfindung sieht besonders bevorzugt vor, eine Temperatur der Kollagenzusammensetzung von mehr als 37° C zu vermeiden.

Besonders bevorzugt werden die temperierten Lösungen erst unmittelbar vor Applikation an den intrakutanen Bestimmungsort vermischt, um dabei die gelierfähige Kollagenzusammensetzung zu bilden, die unmittelbar bei der Applikation beginnt zu gelieren und nach bevorzugt erfolgender Modellierung, in dem Applikationsort ausgeliert, das heißt aushärtet.

Sowohl Kollagenlösung als auch Pufferlösung liegen vor Gebrauch oder Applikation in flüssiger Form vor. Bevorzugt sind Kollagen- und/oder Pufferlösung wässrige Lösungen. Ihre Viskosität erlaubt vorteilhafterweise deren unmittelbare Vermischung ohne zusätzliche denaturierende Maßnahmen wie Erhitzen. Die dynamische Viskosität der Pufferlösung liegt bevorzugt im Bereich derer von Wasser oder leicht beweglichen wässrigen Lösungen, das heißt etwa 1 bis 5 mPa•s.

Kollagenlösung und Pufferlösung werden bevorzugt zunächst voneinander getrennt, bevorzugt in einer mehrkammerigen Spritze, bereitgestellt; sie werden besonders bevorzugt innerhalb der Spritze jeweils getrennt voneinander temperiert und beim Austrag aus der Spritze heraus unmittelbar vermischt. In einer anderen Ausführungsform kann auch vorgesehen sein, Kollagenlösung und Pufferlösung miteinander zu mischen und dann die gelierfähige Kollagenzusammensetzung in dieser vermischten Form zu temperieren. Besonders bevorzugt ist vorgesehen, dass Zusammenbringen und Vermischen von Kollagenlösung und Pufferlösung durch Ausdrücken der Lösungen aus den Kammern der Spritze und durch Zusammenführen der Lösungsströme innerhalb der Spritze in einer der Spritze zugeordneten Mischvorrichtung erfolgt, wobei die frisch hergestellte Kollagenzusammensetzung aus dem Ausgang der Mischvorrichtung austritt.

Die Erfindung sieht bevorzugt vor, dass die im Zusammenhang mit dem offenbarten Verfahren, dem erfindungsgemäßen Kit oder Spritze bereitgestellte, vorzugsweise konzentrierte, Kollagenlösung unmittelbar und ohne denaturierende Schritte aus kollagenhaltigem Gewebe gewonnen wird. In bevorzugter Ausführungsform wird die kollagenhaltige Lösung aus dem kollagenhaltigen Gewebe ohne Einsatz von Enzymen erhalten. In besonders bevorzugter Ausführungsform wird die kollagenhaltige Lösung aus dem kollagenhaltigen Gewebe ohne Einsatz von Alkalien erhalten. In besonders bevorzugter Ausführungsform wird die kollagenhaltige Lösung aus dem kollagenhaltigen Gewebe ohne Einsatz von Enzymen und Alkalien erhalten. In besonders bevorzugter Ausführungsform wird die kollagenhaltige Lösung aus dem kollagenhaltigen Gewebe ohne Einsatz das zu erhaltende Kollagen mechanisch beschädigender Schritte erhalten, z. B. ohne Einsatz von Homogenisierungen, erhalten. Ein bevorzugtes kollagenhaltiges Gewebe sind präparierte Rattenschwanzsehnen. Daraus wird das Kollagen bevorzugt mittels saurer, besonders essigsaurer, Extraktion gewonnen, insbesondere wird das Kollagen allein, das heißt ohne weitere Verfahrensschritte, mittels saurer Extraktion gewonnen.

In besonders bevorzugter Ausführungsform wird eine erfindungsgemäß einzusetzende Kollagenlösung aus Rattenschwänzen gewonnen, indem aus von Haut befreiten Rattenschwänzen Kollagenfasern herausgelöst, in Essigsäurelösung, insbesondere in der Kälte, inkubiert, nicht gelöste Kollagenanteile von gelösten Kollagenanteilen zentrifugiert und abfiltriert, die gelösten Kollagenanteile ausgefällt, diese mit Pufferlösung gespült, eingefroren und gefriergetrocknet werden. Anschließend wird das erhaltene gefriergetrocknete Kollagenpräparat in Essigsäure aufgenommen, so dass ein Kollagengehalt von 8 bis 16 mg/ml, bevorzugt 9 bis 16 mg/ml, insbesondere 10 bis 15 mg/ml, bevorzugt 8 bis 12 mg/ml, insbesondere 10 mg/ml erhalten wird.

In besonders bevorzugter Ausführungsform wird die erfindungsgemäß einzusetzende Kollagenlösung gemäß Beispiel 1, Schritt i), hergestellt.

In besonders bevorzugter Ausführungsform ist die erhaltene kollagenhaltige Lösung, das heißt die Kollagenlösung, vom Grundsatz her, also potentiell, gelierfähig, das heißt kann ausgelieren. In einer bevorzugten Ausführungsform wird die kollagenhaltige Lösung mittels saurer Extraktion aus dem kollagenhaltigen Gewebe erhalten, wobei sie in dieser Ausführungsform ihre tatsächliche Gelierfähigkeit durch Zugabe eines pH-Wert erhöhenden Agens, insbesondere einer Pufferlösung mit neutralisierendem Charakter, erhält.

Als Kollagenlösung wird in bevorzugter Ausführungsform eine saure Kollagenlösung bereitgestellt, insbesondere eine solche, die einen Kollagengehalt (Kollagenkonzentration) von mehr als 8 mg/ml, besonders von etwa 9 mg/ml und mehr oder bevorzugt bis etwa 16 mg/ml, aufweist. In besonders bevorzugter Ausführungsform liegt die Kollagenkonzentration der, vorzugsweise konzentrierten, Kollagenlösung, insbesondere der sauren Kollagenlösung, bei von 8 bis 16 mg/ml, bevorzugt 9 bis 16 mg/ml, insbesondere 10 bis 15 mg/ml, insbesondere 8 bis 12 mg/ml, insbesondere 9 bis 11 mg/ml, vorzugsweise 10 mg/ml. Die, vorzugsweise konzentrierte, Kollagenlösung ist vorzugsweise sauer. Dies insbesondere, um deren Viskosität zu erhalten. Dabei beträgt der pH-Wert der vorliegenden Kollagenlösung (bezogen auf 21° C) pH 6 oder weniger, besonders von pH 5 bis pH 3,5.

In einer besonderen Ausgestaltung enthält die vorliegende Kollagenlösung keine weiteren Zusätze oder Hilfsstoffe wie Zellen, Zellbestandteile, Wachstumsfaktoren wie Cytokine, Immunstimulanzien, Antibiotika oder Stabilisierungsmittel wie Polysaccharide. In alternativer Ausgestaltung ist in der Kollagenlösung mindestens ein solcher Zusatz- oder Hilfsstoff enthalten.

In einer weiteren Ausgestaltung der Erfindung enthält die vorliegende Kollagenlösung keine partikulären Materialien, insbesondere keine Mikrocarrier, Beads (Kügelchen) oder dergleichen.

Um die gelierende Kollagenzusammensetzung zu erhalten, wird die vorliegende, vorzugsweise saure, Kollagenlösung mit einem neutralisierenden Agens, insbesondere Puffer, vermischt. Der neutralisierende Puffer ist im einfachsten Fall eine an sich bekannte Puffersalzlösung, wodurch der pH-Wert der Kollagenzusammensetzung auf einen neutralen Bereich, insbesondere pH 7,0 bis pH 7,5 (bezogen auf 21° C), erreicht wird. Bevorzugt wird eine HEPES-gepufferte Saline mit pH 8,3 eingesetzt, die in an sich bekannter Weise herstellbar ist. Die Pufferlösung dient außerdem dazu, die vorliegende Kollagenlösung zu verdünnen, um die erwünschte Endkonzentration in der gelierenden Kollagenzusammensetzung zu erzielen. Die Pufferzusammensetzung ist je nach vorgesehenem Verhältnis der Mischung mit der vorliegenden Kollagenlösung bevorzugt zumindest zweifach konzentriert (bei 1+1) und maximal zehnfach konzentriert (bei 9+1), bevorzugt fünffach konzentriert (bei 4 + 1).

In einer besonderen Ausgestaltung enthält die Pufferlösung oder die Kollagenlösung keine weiteren Zusätze oder Hilfsstoffe wie Zellen, Zellbestandteile, Wachstumsfaktoren wie Cytokine, Immunstimulanzien oder Stabilisierungsmittel wie Polysaccharide. In alternativer Ausgestaltung sind in der Pufferlösung oder der Kollagenlösung Zellen und gegebenenfalls mindestens ein weiterer Zusatz- oder Hilfsstoff enthalten, die bei dem Vermischen mit der konzentrierten Kollagenlösung eine zellhaltige gelierende Kollagenzusammensetzung bilden, die zu einem zellhaltigen Kollagentransplantat aushärtet. In einer besonderen Variante davon sind die Zellen Fibroblasten, vor allem autologe Zellen oder Stammzellen.

In einer besonders bevorzugten Ausführungsform der vorliegenden Erfindung enthält auch die Pufferlösung keine partikulären Materialien wie Beads (Kügelchen) und/oder Mikrocarrier oder dergleichen.

Bevorzugt ist vorgesehen, Kollagenlösung und Pufferlösung in einem Volumenverhältnis von 1 zu 1 bis 9 zu 1 (Kollagen- zu Pufferlösung) zu vermischen. Bevorzugt sind Mischungsverhältnisse von 4 zu 1, das heißt von vier Teilen Kollagenlösung zu einem Teil Pufferlösung.

Der Kollagengehalt in der hergestellten flüssigen, insbesondere wässrigen, gelierfähigen Kollagenzusammensetzung beträgt bevorzugt mindestens 6 mg/ml oder mehr, besonders von 6 bis 12 mg/ml, insbesondere 6 bis 10 mg/ml. In einer bevorzugten Variante davon beträgt der Gehalt an Kollagen in der gelierfähigen Kollagenzusammensetzung etwa 8 mg/ml.

Bevorzugt wird eine mehrkammerige Spritze eingesetzt. Diese ist besonders bevorzugt eine an sich bekannte Einmalspritze. In einer bevorzugten Variante ist die Spritze mit einem an sich bekannten statischen Mischer als Mischvorrichtung ausgestattet. Der Fachmann kennt daneben andere integrale Mischvorrichtungen, die ein Vermischen von getrennten Lösungen bei der Applikation ermöglichen. In alternativen Ausgestaltungen sind solche anderen Anordnungen ebenfalls Gegenstand der Erfindung.

Werden Kollagenlösung und Pufferlösung in einer mehrkammerigen Spritze bereitgestellt, weist diese besonders bevorzugt jeweils Kammervolumina von etwa 0,1 bis etwa 10 ml auf. Dabei findet das bevorzugte Temperieren der Lösungen bevorzugt innerhalb der Spritze statt, ebenso das Vermischen von Kollagenlösung und Pufferlösung beim Austragen der Lösung aus der Spritze. Bevorzugt ist vorgesehen, dass die Mischvorrichtung der Spritze ein an sich bekannter statischer Mischer ist. Der Fachmann kennt alternative Mischvorrichtungen, die im Zusammenhang mit Spritzen eingesetzt werden können. Der Vorgang des Ausbringens soll je nach Spritzenvolumen und Ausbringgeschwindigkeit etwa 1 bis 60 Sekunden dauern. Die Vermischdauer beträgt für jeden aus den beiden Kammern austretenden infinitesimalen Volumenteil bevorzugt etwa 0,5 bis etwa 2 Sekunden.

Die naszierende Zusammensetzung beginnt unmittelbar zu gelieren und zwar besonders bevorzugt innerhalb von 10 Sekunden nach dem Vermischen, bevorzugt innerhalb von 5 Sekunden. Die gelierende Zusammensetzung ist noch flüssig und vor allem noch fließfähig. Der Geliervorgang ist abgeschlossen, wenn die Kollagenzusammensetzung zu einer festen Biomatrix aushärtet. Das Aushärten zu einer Biomatrix ist bevorzugt innerhalb von 2 bis 4 Minuten abgeschlossen. Bevorzugt findet so die Aushärtung der in die Lederhaut applizierten zunächst noch fließfähigen Kollagenzusammensetzung erst in dieser selbst statt, sodass dort, sofern gewünscht, ein Formen, das heißt dreidimensionales Modellieren, erfolgen kann, bevor der Gelierprozess abgeschlossen ist.

Die Erfindung stellt in diesem Zusammenhang auch eine fertig gefüllte Spritze, insbesondere in Form einer Einmalspritze, bereit, wobei die Spritze mindestens zwei getrennte Kammern enthält, die in einer der Spritze zugeordneten Spritzvorrichtung münden, durch die der Inhalt jeder Kammer bevorzugt simultan abgegeben werden kann, und wobei die Spritze eine Nadel aufweist, deren Außendurchmesser 0,3 bis 0,4 mm, vorzugsweise 0,3 mm, vorzugsweise 0,32 bis 0,38 mm, insbesondere 0,35 mm, beträgt. Erfindungsgemäß ist die Spritze zumindest ferner dadurch gekennzeichnet, dass mindestens eine erste Kammer mit der flüssigen konzentrierten Kollagenlösung und mindestens eine davon getrennte zweite Kammer mit der Pufferlösung gefüllt sind. Daneben können weitere solche Kammern an der Spritze vorgesehen sein, die mit Zusatz- oder Hilfsstoffen gefüllt sein können.

Die zumindest mit erfindungsgemäßer Kollagenlösung und Pufferlösung gefüllte Spritze stellt zusammen mit einer die vorliegende Indikation zur intrakutanen Applikation zur Therapie von Hautdefekten, z. B. bei Hauterkrankungen oder Hautalterungserscheinungen, darstellenden Packungsbeilage einen Kit dar, der zur Herstellung einer unmittelbar gelierenden Kollagenzusammensetzung eingesetzt werden kann.

Erfindungsgemäß enthält ein derartiger Kit einen Behälter enthaltend eine Kollagenlösung und einen Behälter enthaltend eine neutralisierende Pufferlösung.

Daneben ist die Erfindung nicht auf die Verwendung einer gefüllten mehrkammerigen Spritze beschränkt. Es werden auch Ausgestaltungen bereitgestellt, die eine getrennte Lagerung von konzentrierter Kollagenlösung und Pufferlösung und die anschließende unmittelbare Vermischung dieser Lösungen zur Herstellung der gelierenden Kollagenzusammensetzung erlauben. Eine alternative Ausgestaltung ist insbesondere eine mehrkammerige Mischkapsel zur einmaligen Verwendung, die im Zusammenhang mit einem an sich bekannten Kapselmischer eingesetzt werden kann.

Gegenstand der Erfindung ist auch die Verwendung der erfindungsgemäßen gefüllten Spritze oder des erfindungsgemäßen Kits zur Anwendung zur medizinischen Therapie. Eine besondere Anwendung ist die prophylaktische oder therapeutische Behandlung von Hautdefekten, insbesondere von Hauterkrankungen und Hautalterungserscheinungen des menschlichen oder tierischen Körpers, durch intrakutanes Auffüllen des Kollagengerüstes im Bindegewebe der Haut des Körpers.

Im Zusammenhang mit der vorliegenden Erfindung werden unter Hautdefekten insbesondere Hauterkrankungen und Hautalterungserscheinungen verstanden. Hautdefekte im Sinne der vorliegenden Erfindung können auch im Rahmen oder im Umfeld von Hauterkrankungen und Hautalterungserscheinungen auftretende Symptome oder Begleitsymptome sein.

Im Zusammenhang mit der vorliegenden Erfindung werden unter Hautdefekten sämtliche vom Normalzustand der Haut eines gesunden menschlichen oder tierischen Körpers abweichende Hauterscheinungsformen verstanden, die sich durch intrakutanes Auffüllen des Kollagengerüstes der Haut prophylaktisch verhindern, das heißt vorbeugen, oder therapieren, insbesondere reparieren, lassen. Derartige Hautdefekte, z. B. Hauterkrankungen oder Hautalterungserscheinungen, können auf Verletzungen, auf Alterungsprozessen, auf Einwirkungen externer Einflüsse, zum Beispiel Strahlung, Hitze, Feuer, Flüssigkeiten oder dergleichen, beruhen. Unter derartigen Hautdefekten werden auch Hautdefekte kosmetischer Natur verstanden, also solche, die eher optischer und weniger gesundheitlicher Natur sind. Hautdefekte kosmetischer Natur können auch Erscheinungsformen der Haut sein, die vom Patienten oder seiner Umgebung als verbesserungs-, insbesondere verschönerungsfähig, identifiziert werden, ohne dass eine gesundheitliche Notwendigkeit für eine Therapie vorliegt. Insbesondere betrifft die vorliegende Erfindung Hautdefekte in Form von Falten.

Die Erfindung wird durch die Figuren und die nachfolgenden Ausführungsbeispiele näher beschrieben, ohne dass diese beschränkend zu verstehen wären.

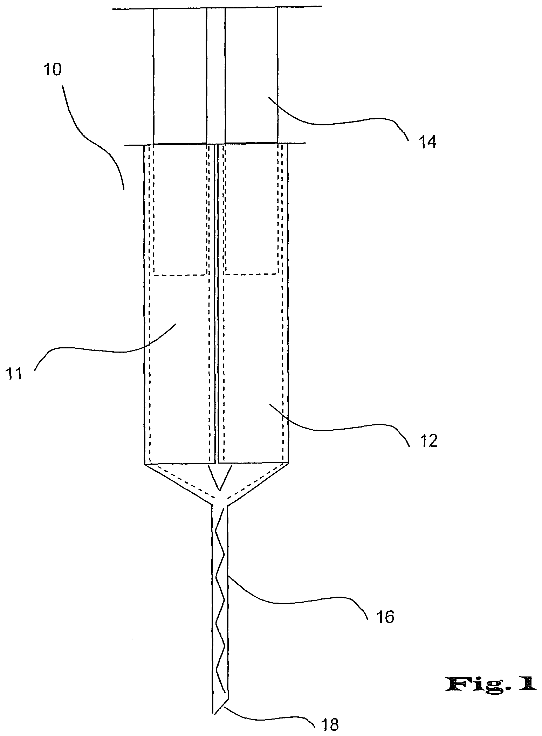

Figur 1 zeigt eine schematische Darstellung einer Ausgestaltung der erfindungsgemäßen gefüllten Spritze als Mittel zur Durchführung des offenbarten Verfahrens.
   In der dargestellten Ausführung sind in einem integralen Behälter zwei getrennte parallele zylindrische Kammern (11, 12) zur Aufnahme der Kollagenlösung einerseits und Pufferlösung andererseits ausgebildet. Beide Kammern münden vorderseits in einen gemeinsamen Austrittskanal (18) eines statischen Mischers (16), wobei in dem Mischer eine Mischschikane vorliegt. Dem Austrittskanal (18) ist eine Nadel mit einem Außendurchmesser von 0,3 mm vorgeschaltet. Zum Entleeren der Kammern (11, 12) zur Applikation und Herstellung der Kollagenzusammensetzung sind gekoppelte Spritzenstempel (14) vorgesehen. Diese bilden die rückseitige Begrenzung des Volumens der Kammern: in an sich bekannter Weise kann durch Druckbeaufschlagung der Stempel (14) der Inhalt simultan aus beiden Kammern (11, 12) ausgedrückt werden. Je nach Verhältnis des baulich vorgegebenen Volumens der Kammern (11, 12) findet beim Ausdrücken eine gleichmäßige Vermischung der sich in den Kammern (11, 12) befindlichen Flüssigkeit in diesem Volumenverhältnis statt. In der dargestellten Ausgestaltung ist das Volumenverhältnis der Kammern (11, 12) 1:1.
Figur 2 zeigt die Ergebnisse rheologischer Untersuchungen an Kollagengelen: elastischer Modulus in Abhängigkeit von der Testfrequenz (Mittelwerte und Standardabweichungen) bei unterschiedlich vortemperierten Zweikammerspritzen.
Figur 3 zeigt eine fotografische Darstellung einer Zweikammerspritze, wie sie schematisch in Figur 1 dargestellt ist und in Beispiel 4 eingesetzt wurde.
Figur 4 zeigt die Ergebnisse rheologischer Untersuchungen zur Bestimmung der Viskosität von erfindungsgemäß einzusetzenden flüssigen Kollagenzusammensetzungen.
Die Figuren 5 bis 10 zeigen fotografisch den Ablauf einer erfindungsgemäß ermöglichten Therapie eines Hautdefekts an einem Schweinehautmodell mit intrakutanem Applizieren und anschließendem Modellieren vor dem Aushärten des injizierten gelierfähigen Kollagens.

### Beispiel 1: Herstellung einer unmittelbar gelierenden flüssigen Kollagenzusammensetzung

i) Zur Herstellung einer konzentrierten Kollagenlösung werden Rattenschwänze bei etwa minus 20° C gelagert und anschließend für einige Minuten in etwa 70 %igem Alkohol oberflächlich desinfiziert. Es wird die Haut abgezogen und die einzelnen Kollagenfasern herausgelöst. Die Kollagenfasern werden nochmals in Alkohol oberflächlich desinfiziert, danach mit PBS gewaschen, anschließend in eine etwa 0,1 %ige (0,5 mol/l) Essigsäurelösung überführt und dort inkubiert. Die Kollagenfasern werden in der Essigsäurelösung für einen Zeitraum von mindestens 7 Tagen in der Kälte (bei etwa 0 bis 4° C) gerührt. Nach Abtrennen der nichtgelösten Kollagenteile am Ende der Inkubationszeit wird das Kollagen filtriert und ausgefällt. Das Präzipitat wird mit Pufferlösung gespült, eingefroren und anschließend gefriergetrocknet. Das gefriergetrocknete Kollagen wird in 0,1 % Essigsäure definiert aufgenommen, sodass ein Kollagengehalt von 8 bis 16 mg/ml, insbesondere 9 bis 16 mg/ml erhalten wird. Der pH-Wert der konzentrierten Kollagenlösung beträgt etwa 4,0.
ii) Zur Vermischung von vier Teilen Kollagenlösung mit einem Teil neutralisierender Pufferlösung (4+1) wird eine fünffach konzentrierte Pufferlösung angesetzt. Als fünffach konzentrierte Pufferlösung wird dazu insbesondere eine Lösung von 35,6 g NaCl in 937,5 ml Reinstwasser mit 62,5 ml 3 mol/l HEPES-Lösung angesetzt. Vor Gebrauch wird die Pufferlösung mit NaOH auf pH 8,3 eingestellt.
   Kollagenlösung und Pufferlösung werden in jeweils getrennte Kammern einer Mehrkammerspritze, die ein Kammervolumenverhältnis von 1:4 aufweist, eingefüllt und bis zu deren weiteren Verwendung in der Kälte, bei etwa -15° C oder kälter, gelagert.
iii) Zur Herstellung einer kollagenhaltigen Biomatrix wird die Mehrkammerspritze kurzfristig in einen Wärmeschrank oder ein Wasserbad gelegt, wodurch Pufferlösung und Kollagenlösung auf eine Temperatur von etwa 30° C, erwärmt werden. Zum Vermischen der beiden Lösungen werden diese über die gekoppelten Spritzenkolben aus der Mehrkammerspritze gedrückt. Dabei werden beide Lösungen über die mit den Kammern verbundene Mischvorrichtung geführt. Aus der Spritze und tritt die vermischte, unmittelbar gelierende Kollagenzusammensetzung aus. Diese wird in noch flüssiger Form in eine Gießform eingefüllt. Nach dem Ausbringen füllt die Kollagenzusammensetzung die Gießform völlig aus, und innerhalb weniger Minuten härtet sie zu einer festen kollagenhaltigen Biomatrix aus. Bei einem Kollagengehalt der durch die Vermischung mit Pufferlösung entstehenden flüssigen Kollagenzusammensetzung von etwa 8 mg/ml und einer Temperatur von etwa 30° C härtet diese innerhalb von 2 Minuten vollständig aus.

### Beispiel 2: Gelierung einer Kollagenzusammensetzung

Die gemäß Beispiel 1 hergestellten Kollagenzusammensetzungen werden rheologischen Untersuchungen zur Bestimmung des Elastizitätsmoduls unterzogen. In Zweikammerspritzen (z. B. Firma Medmix Systems, CH) werden Kollagenlösung (10 mg/ml) und Pufferlösung gemäß Beispiel 1 gefüllt (Kollagenkammer: 4 Teile Kollagenlösung, 2 ml; Pufferkammer: 1 Teil fünffach konzentrierter Puffer, 0,5 ml) und eingefroren.

Dem einstündigen, schonenden Auftauen bei Raumtemperatur (20,5 °C) folgt eine zehnminütige Temperierung der Spritzen im Wasserbad bei unterschiedlichen Temperaturen im Bereich von 20 bis 40 °C (Tabelle 1).

**Tabelle 1:**

| Temperatur Soll [°C] (Temperaturbereich Ist [°C]) | | Anzahl der untersuchten Spritzen |
|---|---|---|
| 20 | (19 - 20) | 3 |
| 30 | (30 - 31) | 4 |
| 37 | (36,5 - 37) | 4 |
| 38 | (38 - 39) | 3 |
| 40 | (40 - 41) | 4 |

Zum Mischen beider Komponenten nach der Temperierung wird der Verschluss der Spritze durch einen Mischadapter ausgetauscht und der Inhalt vorsichtig in ein Well einer 12-Well-Gewebekulturplatte abgegeben, dabei werden die ersten zwei austretenden Tropfen verworfen.

Die vollständige Gelierung der Kollagenzusammensetzung (8 mg/ml Kollagen), das heißt Aushärtung zu einem festen Gel, erfolgte in 15 min bei 20,5 °C. Anschließend erfolgt eine visuelle Beurteilung der Konsistenz (Tabelle 2).

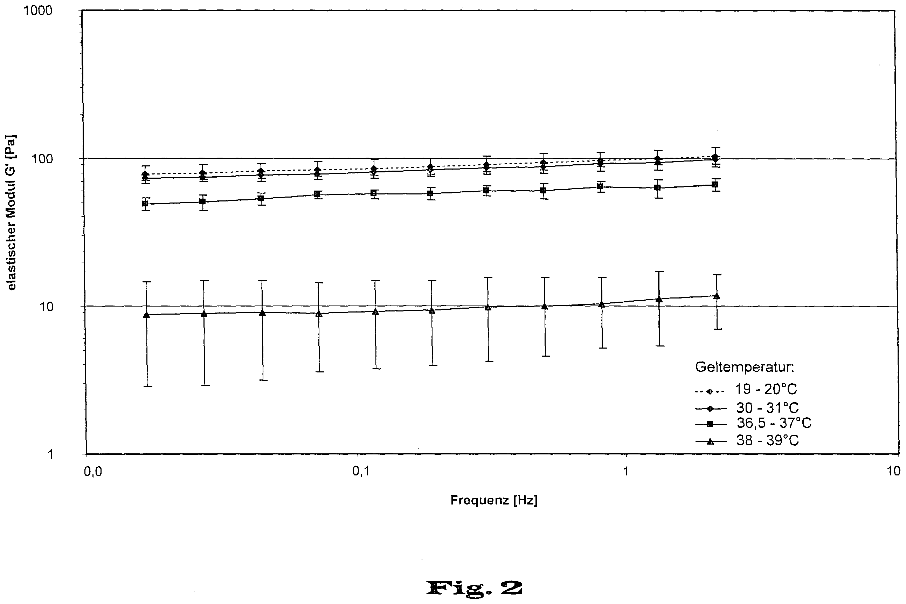

Anschließend werden die elastischen Module der Gele außerhalb des Wells durch Frequenzverfahren ermittelt (Bohlin CVO R 150, Fa. Malvern Instruments GmbH, DE) (Figur 2).

**Tabelle 2:**

| Temperatur Soll [°C] (Temperaturbereich Ist [°C]) | | Konsistenz der Gele nach 15 min |
|---|---|---|
| 20 | (19 - 20) | fest |
| 30 | (30 - 31) | fest |
| 37 | (36,5 - 37) | fest |
| 38 | (38 - 39) | halbfest |
| 40 | (40 - 41) | flüssig |

Figur 2 ist zu entnehmen, dass die höchsten ermittelten Mittelwerte des elastischen Moduls in einem Temperaturbereich von 20 und 30 °C liegen. Die Kurve des auf 37 °C erwärmten Kollagens verläuft etwas tiefer, jedoch haben auch diese Gele bei visueller Betrachtung eine feste Konsistenz gezeigt. Bei Erwärmung auf 38 °C waren die Gele nur halbfest und wiesen bei der Oszillationsmessung sehr niedrige Werte mit großen Schwankungen auf. Das 40 °C warme Kollagen geliert nicht mehr.

### Beispiel 3: Rheologische Messungen zur Bestimmung der Viskosität

Geräte:
- Rheometer System C-VOR 150 CE/WIN inklusive Luftfiltereinheit, Fa. Bohlin Pforzheim
- Peltier System 180° für CVO für Temperaturen von - 30 °C bis 180 °C
- Wasserkühleinheit
- Kegel-Platte-Messsystem CP 4°/40 mm
- Computer: siehe 06SOP-01-010
   Software: Softwaremodul Viskosimetrie

### Viskositätsmessung von Kollagen

Die Messungen erfolgen bei einer Scherrate von 0,32 bis 56,23 s-1. Gemessen wird die Viskosität des Kollagens. Es wird eine Reproduzierbarkeitsmessung nach Reinigung der Platten und Neubefüllung durchgeführt.

Im ersten Schritt wird im Rheometer die Dichte des Materials mit 1000.000 kg/m³ (ungefähre Dichte des Kollagens bei 23°C) eingeben. Anschließend wird die Probe (min. 2 mL Kollagen) auf die untere Platte zentrisch aufgeben und die Messgeometrie nach unten gefahren (automatisches Abstoppen auf voreingestellten Spaltabstand von 150 µm).

Die Viskositätsmessung erfolgt mit folgenden Parametern:
Vorscherung: 100 s-1
Anwendungsdauer: 10 s
Ausgleichszeit: 30 s
Viskosimetrie-Modus: Scherratentabelle 0,1 bis 100 s-1 (25 Werte, logarithmisch geordnet)
Verzögerungszeit: 5 s konstant
Integrationszeit: 5 s

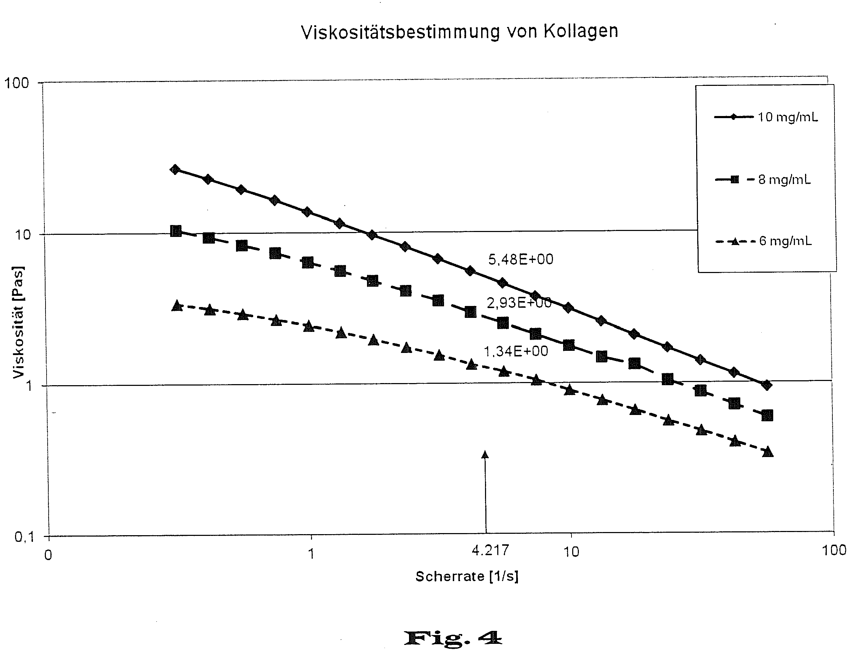

Die Rohdaten der Scherratenmessung von 0,32 bis 56,23 s-1 werden für die Erstmessung und die Reproduzierbarkeitsmessung in einer Exceltabelle zusammengefasst und graphisch dargestellt. In der Figur 4 sind die Ergebnisse für Kollagenkonzentrationen von 6, 8 und 10 mg/ml Kollagen dargestellt, wobei die Kollagenlösungen gemäß Beispiel 1 hergestellt wurden.

Bei einer Scherrate von 4,217/s ergibt sich für die 6 mg/ml Kollagenlösung eine Viskosität von 1,34 Pa x s, für 8 mg/ml Kollagenlösung eine Viskosität von 2,93 Pa x s und für die 10 mg/ml Kollagenlösung eine Viskosität von 5,48 Pa x s.

### Beispiel 4: Applikation in Schweinehaut

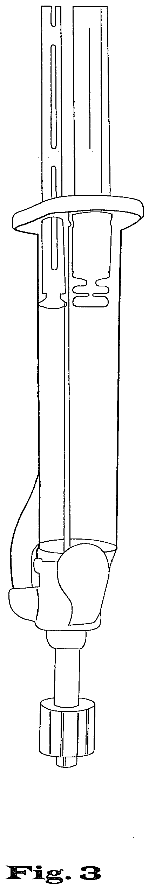

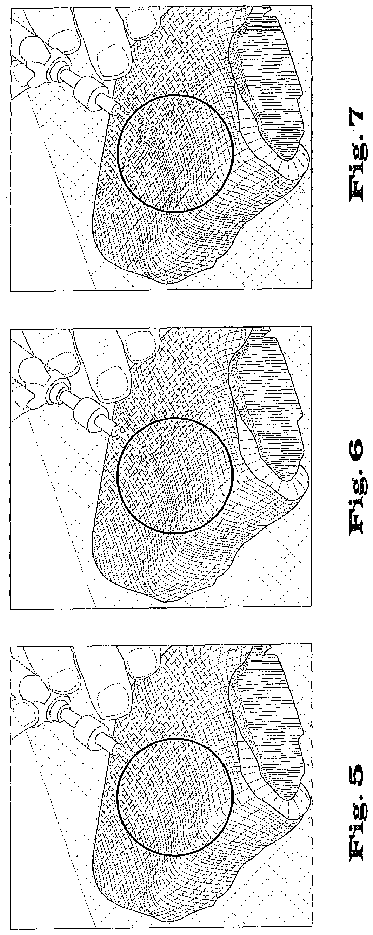

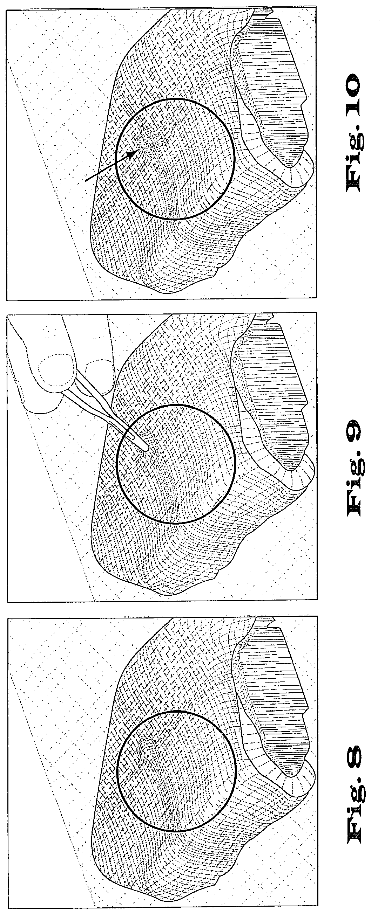

Die Einspritzbarkeit der gemäß dem offenbarten Verfahren bereitgestellten flüssigen gelierfähigen Kollagenzusammensetzung durch eine 30 Gauge-Nadel wurde für das flüssige gelierfähige Kollagen-Puffergemisch gemäß Beispiel 1 mit einer nativen Kollagenkonzentration von 8 mg/ml an einer Schweinehaut untersucht. Die Proben des 8 mg/ml natives Kollagen-Puffergemisch wurden über eine Zweikammerspritze (Figur 1 und 3; Kammer 11 gefüllt mit 2 ml einer 10 mg/ml Kollagenlösung, Kammer 12 gefüllt mit 0,5 ml einer Pufferlösung) intrakutan in eine Schweinehaut gespritzt (Figur 5 bis 7). Anschließend wurde die intrakutan applizierte noch flüssige Kollagen-Pufferlösung (Figur 8) von außen modelliert (Figur 9). In Figur 10 ist das Ergebnis des modellierten Gels dargestellt. Die Kollagen-Pufferlösung konnte über die 30 Gauge-Nadel intrakutan injiziert werden, ist dort geliert und hat nach Modellierung ihre Form beibehalten.

## Patentansprüche

1. Kit zur Verwendung in einem therapeutischen Verfahren zum intrakutanen Auffüllen des Kollagengerüsts in Bindegewebe der Haut eines Patienten, umfassend mindestens einen Behälter enthaltend eine Kollagenlösung, mindestens einen Behälter enthaltend eine neutralisierende Pufferlösung und eine Spritze geeignet zum intrakutanen Injizieren der Kollagen- und Pufferlösung, wobei die Nadel der Spritze einen Durchmesser von 0,3 bis 0,4 mm aufweist.

2. Kit nach Anspruch 1, wobei die Spritze mindestens zwei getrennte als Kammern ausgeführte Behälter aufweist, die in Fluidverbindung mit einer in der Spritze angeordneten Mischvorrichtung und der Nadel der Spritze stehen, wobei mindestens eine erste Kammer mit der Kollagenlösung und mindestens eine davon getrennte zweite Kammer mit der neutralisierenden Pufferlösung gefüllt ist.

3. Kit nach Anspruch 2, wobei die Mischvorrichtung ein statischer Mischer ist.

4. Spritze zur Verwendung in einem therapeutischen Verfahren zum intrakutanen Auffüllen des Kollagengerüsts in Bindegewebe der Haut eines Patienten, enthaltend mindestens zwei getrennte als Kammern ausgeführte Behälter, die in Fluidverbindung mit einer in der Spritze angeordneten Mischvorrichtung und der Nadel der Spritze stehen, wobei mindestens eine erste Kammer mit einer Kollagenlösung und mindestens eine davon getrennte zweite Kammer mit einer neutralisierenden Pufferlösung gefüllt ist, und wobei die Nadel der Spritze einen Durchmesser von 0,3 bis 0,4 mm aufweist.

5. Kit oder Spritze nach einem der vorhergehenden Ansprüche, wobei der pH-Wert der Kollagenlösung bezogen auf 21°C 6 oder weniger beträgt.

6. Kit oder Spritze nach einem der vorstehenden Ansprüche, wobei die Kollagenlösung durch saure Extraktion ohne Einsatz enzymatischer Aktivität aus kollagenhaltigem Gewebe, insbesondere Rattenschwänzen, hergestellt wurde.

7. Kit oder Spritze nach einem der vorhergehenden Ansprüche, wobei die Kollagenlösung, die flüssige gelierfähige Kollagenzusammensetzung oder beides zellfrei ist.

## Claims

1. Kit for use in a therapeutic method for intracutaneous filling of the collagen structure in connective tissue of a patient's skin, comprising at least one container containing a collagen solution, at least one container containing a neutralizing buffer solution, and a syringe suited for intracutaneous injection of the collagen and buffer solution, wherein the needle of the syringe has a diameter of 0.3 to 0.4 mm.

2. Kit according to claim 1, wherein the syringe has at least two separate containers configured as chambers, which are in fluid connection with a mixing device arranged in the syringe and with the needle of the syringe, wherein at least a first chamber is filled with the collagen solution and at least a second chamber separate therefrom is filled with the neutralizing buffer solution.

3. Kit according to claim 2, wherein the mixing device is a static mixer.

4. Syringe for use in a therapeutic method for intracutaneous filling of the collagen structure in connective tissue of a patient's skin, comprising at least two separate containers configured as chambers, which are in fluid connection with a mixing device arranged in the syringe and with the needle of the syringe, wherein at least a first chamber is filled with a collagen solution and at least a second chamber separate therefrom is filled with a neutralizing buffer solution, and wherein the needle of the syringe has a diameter of 0.3 to 0.4 mm.

5. Kit or syringe according to any one of the preceding claims, wherein the pH of the collagen solution, based on a temperature of 21°C, is 6 or lower.

6. Kit or syringe according to any one of the preceding claims, wherein the collagen solution was produced from collagen-containing tissue, in particular rat tails, by acid extraction without using enzymatic activity.

7. Kit or syringe according to any one of the preceding claims, wherein the collagen solution, the liquid gellable collagen composition, or both are cell-free.

## Revendications

1. Kit pour une utilisation dans un procédé thérapeutique pour le comblement intracutané de la structure de collagène dans le tissu conjonctif de la peau d'un patient, englobant au moins un récipient contenant une solution de collagène, au moins un récipient contenant une solution tampon neutralisante et une seringue appropriée à l'injection intracutanée de la solution de collagène et de la solution tampon, l'aiguille de la seringue présentant un diamètre de 0,3 à 0,4 mm.

2. Kit conformément à la revendication n°1, la seringue présentant au moins deux récipients séparés, réalisés sous forme de compartiments, qui sont en liaison fluidique avec un dispositif de mélange placé dans la seringue et l'aiguille de la seringue, dans lequel au moins un premier compartiment est rempli de la solution de collagène et au moins un deuxième compartiment séparé de ce dernier est rempli de la solution tampon neutralisante.

3. Kit conformément à la revendication n°2, le dispositif de mélange étant un mélangeur statique.

4. Seringue pour une utilisation dans un procédé thérapeutique pour le comblement intracutané de la structure de collagène dans le tissu conjonctif de la peau d'un patient, contenant au moins deux récipients séparés, réalisés sous forme de compartiments, qui sont en liaison fluidique avec un dispositif de mélange placé dans la seringue et l'aiguille de la seringue, dans laquelle au moins un premier compartiment est rempli d'une solution de collagène et au moins un deuxième compartiment séparé de ce dernier est rempli de la solution tampon neutralisante et dans laquelle l'aiguille de la seringue présente un diamètre de 0,3 à 0,4 mm.

5. Kit ou seringue conformément à l'une des revendications précédentes, la valeur du pH de la solution de collagène s'élevant à 6 ou moins en référence à 21°C.

6. Kit ou seringue conformément à l'une des revendications précédentes, la solution de collagène étant produite par une extraction acide sans application d'une activité enzymatique issue du tissu contenant le collagène, en particulier des queues de rats.

7. Kit ou seringue conformément à l'une des revendications précédentes, la solution de collagène, la composition de collagène gélifiable liquide ou les deux étant non- cellulaires.
